Europäisches Patentamt

European Patent Office

Office européen des brevets

(11) Publication number: **0 410 002 B1**

## EUROPEAN PATENT SPECIFICATION

(45) Date of publication of patent specification: **22.11.95**  (51) Int. Cl.⁶: **A61K 31/725**

(21) Application number: **90902826.8**

(22) Date of filing: **09.02.90**

(86) International application number:
**PCT/JP90/00159**

(87) International publication number:
**WO 90/09181 (23.08.90 90/20)**

(54) **ANTI-HIV DRUG.**

(30) Priority: **10.02.89 JP 31581/89**

(43) Date of publication of application:
**30.01.91 Bulletin 91/05**

(45) Publication of the grant of the patent:
**22.11.95 Bulletin 95/47**

(84) Designated Contracting States:
**AT BE CH DE DK ES FR GB IT LI LU NL SE**

(56) References cited:
EP-A- 0 295 956     JP-A- 0 002 215
JP-A- 1 100 127     JP-A- 1 103 601
JP-A- 1 132 526     JP-A- 1 165 525
JP-A- 1 199 915     JP-A- 1 230 519
JP-A- 1 275 532     JP-A- 1 287 031
JP-A- 1 313 433     JP-A- 6 345 223
JP-A- 6 413 026     JP-A- 6 425 724
JP-A- 6 450 821     JP-A- 6 452 723
JP-A-63 128 001     JP-A-63 253 026
JP-B- 6 225 126     JP-B- 6 348 849
US-A- 4 840 341

(73) Proprietor: **TAIHO PHARMACEUTICAL COM-
PANY LIMITED**
**1-27, Kandanishiki-cho**
**Chiyoda-ku**
**Tokyo-to (JP)**

Proprietor: **Taiho Fine Chemical Co., Ltd.**
**22, 200-banchi, Aza Toyohara, Oaza**
**Motohara**
**Kamikawamachi, Kodama-gun**
**Saitama 376-02 (JP)**

(72) Inventor: **HOSHINO, Hiroo**
**1-14-5, Heiwamachi**
**Maebashi-shi**
**Gunma 371 (JP)**

(74) Representative: **VOSSIUS & PARTNER**
**Postfach 86 07 67**
**D-81634 München (DE)**

CA112(3):16025d (Ucla Symp. Mol. Cell. Biol., New Ser., 100 (Mech. Action Ther. Appl. Biol. Cancer Immune Defic. Disord., 331-41 (1989) MITSUYA, HIROAKI; LOONEY, DAVID J.; KUNO, SACHIKO; UENO, RYUJI; WONG-STAAL, FLOSSIE; BRODER, SAMUEL: Abstract of "Inhibition of Virion Binding to CD4+ Cells: Suppression of Human Immunodeficiency Viruses by Anionic Polysaccharides".)

CA112(1):258w (Experientia, 45 (10), 996-8 (1989) SUGAWARA, I.; ITOH, W.; KIMURA, S.; MORI S.; SHIMADA, K.: Abstract of "Further Characterization of Sulfated Homopolysaccharides as Anti-Hiv Agents".)

CA111(25):224861e (Chem. Pharm. Bull., 37 (9), 2410-12 (1989) HIRABAYASHI, K.; IWATA, S.; ITO, M.; SHIGETA, S.; NARUI, T.; MORI, T.; SHIBATA, S.: Abstract of "Inhibitory Effect of a Lichen Polysaccharide sulfate, GE-3-S, on the Replication of Human Immunodeficiency Virus (HIV) in Vitro".)

CA111(23):208773x (J. Acquired Immune Defic. Sundr., 2 (5), 441-7 (1989) TOCHIKURA, T. S.; NAKASHIMA, H.; YAMAMOTO, N.:Abstract of "Antiviral Agents with Activity Against Human Retroviruses".)

CA111(15):132308b (J. Immunol., 143(4), 1149-54 (1989) LEDERMAN, S.; GULICK, R.; CHESS, L.: Abstract of "Dextran sulfate and Heparin Interact with CD4 Molecules to Inhibit the Binding of Coat Protein (gp120) of HIV".)

CA110(9):72540p (Antiviral Res., 10(1-3), 107-15 (1988) NGAN, FUNG; CHANG, R. SHIH-MAN; TABBA, HANI D.; SMITH, KEVIN M.: Abstract of "Isolation, Purification and Partial characterization of an Active Anti-HIV Compound from the Chinese Medicinal Herb Violayedoensis".)

CA110(23):205159v (Eur. J. Clin. Micrbiol. Infect. Dis., 8(2), 171-3 (1989) ITO, M.; BABA, M.; HIRABAYASHI, K.; Matsumoto, T.; SUZUKI, M.: SUZUKI, S.; SHIGETS, S.; DE CLERCO, E.: Abstract of "Invitro Activity of Mannan Sulfate, a Novel Sulfated Polysaccharide, Against Human Immunodeficiency Virus Type 1 and other Enveloped Viruses".)

CA110(7):50810z (Antiviral Res., 9(6), 335-43 (1988) BABA, M.; NAKAJIMA, M.; SCHOLS,

DOMINIOUE; PAUWELS, R.; BALZARINI. J.; DE CLERCO, E.: Abstract of "Pentosan Polysulfate, a Sulfated Oligosaccharide, is a Potent and Selective Anti-HIV Agents in Vitro")

CA110(3):18171f (J. Infect. Dis., 158(5), 1084-7 (1988) BAGASRA, O.; LISCHNER, H. W.: Abstract of "Activity of Dextran Sulfate and other Polyanionic Polysaccharides Against Human Immodeficiency Virus".)

CA109(19):163104v (Biochem. Pharmacol., 37(15), 2887-91 (1988) YOSHIDA, O.; MAKASHIMA, H.; YOSHIDA, T.; KANEKO, Y.; YAMAMOTO, I.; MATSUZAKI, K.; URYU, T.; YAMAMOTO, N.: Abstract of "Sulfation of the Immunomodulating Polysaccharide Lentinan: a Novel Strategy for Antivirals to Human Immunodeficiency Virus (HIV)".)

CA109(19):162907x (Jpn. J. Exp. Med., 58(3), 145-51 (1988) MIZUMOTO, K.; SUGAWARA, I.; ITO, W.; KODAMA, T.; HAYAMI, M.; MORI, S.: Abstract of "Sulfated Homopolysaccharides with Immunodulating Activities are more Potent Anti-HTLV-III Agents than Sulfated Hetero Polysaccharides".)

CA109(17):142064s (Proc. Natl. Acad. Sci. U.S.A., 85(16), 6132-6 (1988) BABA, M.; PAUWELS, R.; BALZARINI, J.; ARNOUT, J.; DESMYTER, J.; DE CLERCO, E.: Abstract of "Mechanism of Inhibitory Effect of Dextran Sulfate and Hepain on Replication of Human Immunodeficiency Virus in Vitro")

CA109(11):89558c (Arch. Aids Res., 1(1), 45-56 (1987) YAMAMOTO, N.; NAKASHIMA, H.; YOSHIDA, O.; KANEKO, Y.; MATSUZAKI, K.; URYU, T.: Abstract of "Effect of the Sulfated Polysaccharides on HIV: a Novel Strategy of Chemical Modification for HIV Antivirals".)

CA108(11):87640d (Jpn. J. Cancer Res. (GANN), 78(11), 1164-8 (1987) NAKASHIMA H.; YOSHIDA, O.; TOCHIKURA, T. S.; YOSHIDA, T.; MIMURA, T.; KIDO, Y.; MOTOKI, Y.; KANEKO, Y.; URYU, T.; YAMAMOTO, N.: Abstract of "Sulfation of Polysaccharides generates potent and selective inhibitors of human immunodeficiency virus infection and replication in vitro"

CA108(3):18337y (Antimicrob. Agents Chemother.. 31(10), 1524-8 (1987) NAKASHIMA, H.; KIDO, Y.; KOBAYASHI, N.; MOTOKI, Y.; NEUSHUL, M.; YAMAMOTO, N.:

Abstract of "Purification and Characterization of an Avian Myeloblastosis and Human Immunodeficiency Virus Reverse Transcriptase Inhibitor, Sulfated Polysaccharides Extracted from Sea Algae".)

CA99(3):19901k (Yaoxue Xuebao, 18(3), 203-8 (1982) FAN, HUIZENG; CHEN, JUDI; LU, PEIHONG; HAO, XIAOGE; LE, HAITANG: Abstract of "Acidic Polysaccharides from Holothuria Leucospilota (Brandt)".)

CA94(8):52763m (Yao Hsueh Hsueh Pao,

15(5), 263-70 (1980) FAN, HUI-ZENG; CHEN, JU-DI; LIN, KE-ZHONG: Abstract of "Isolation of an Acidic Mucopolysaccharide from Stichopus Japonicus Selenka and some of its Physical and Chemical Properties".)

CA84(25):175525m (Chem. Pharm. Bull., 24(2), 275-84 (1976) KITAGAWA, ISAO; SUGAWARA, TAMIO; YOSIOKA, ITIRO: Abstract of "Saponin and Sapogenol. XV. Antifungal Glycosides from the Sea Cucumber Stichopus Japonicus Selenka. (2). Structures of Holotoxin A and Holotoxin B")

3

**Description**

The present invention relates to the use of sulfated polysaccharides for the preparation of a novel anti-HIV agent effective against a human immunodeficiency virus (hereinafter referred to as "HIV" throughout the specification).

Acquired immune deficiency syndrome or AIDS is a grave immunodeficiency syndrome caused by the infection of HIV [human immunodeficiency virus; Nature, 321, 10 (1986)]. In view of a very high mortality from this disease, to cope with HIV infection and AIDS has become a serious social issue.

Known anti-HIV agents currently accepted as effective in clinical use include azidothymidine (AZT), which has an inhibitory activity against reverse transcriptase.

However, azidothymidine (AZT) used as an anti-HIV agent gives an unsatisfactory clinical effectiveness. Further, azidothymidine produces serious side effects such as disorders of bone marrow (hematopoietic tissues) and neurological complaints such as headache, convulsion, etc. Since the gene of HIV in the form of a provirus infiltrates itself into the infected cell's chromosomes as in the state of hereditary diseases, it is inevitably necessary to administer a pharmaceutical preparation for a long term. Accordingly a serious obstacle is posed by the side effects of AZT used as an anti-HIV agent.

In the present situation, it is desired to develop a novel medicament effective against HIV infection and AIDS, substantially free of the above side effects and administrable over a long period of time.

Chem. Abstr. 99, 19901k (1983), corresponding to Yaoxue Xuebao, 1983, 18 (3), 203-8, and Chem. Abstr. 94, 52763m (1981), corresponding to Yao Hsueh Hsueh Pao, 1980, 15 (5), 263-70, describe sulfated polysaccharides from sea cucumber having antitumor activity.

JP-A 63045223 discloses anti-HIV activity of sulfated polysaccharides whereby the sulfated polysaccharides are obtained from plants or microorganisms and contain fucose and glucuronic acid or are obtained from animals and contain galactosamine and glucuronic acid.

The present inventor conducted extensive research to develop a compound significantly efficacious against HIV, and found that sulfated polysaccharides derived from sea cucumbers and/or a pharmaceutically acceptable salt thereof have a remarkable anti-HIV activity and can be safely administered to patients over a long period of time. The present invention has been accomplished based on these novel findings.

It is therefore the subject matter of the present invention to provide the use of a sulfated polysaccharide derived from a sea cucumber or a pharmaceutically acceptable salt thereof for the preparation of a pharmacological composition for treating diseases caused by HIV infection, said sulfated polysaccaride consisting of galactosamine, glucuronic acid, fucose and sulfate.

The sulfated polysaccharide derived from a sea cucumber (such polysaccharide will be hereinafter referred to as "FGAG") for use in the invention is one extracted from the body wall of holothurians and a relative prepared by chemical conversion of FGAG.

FGAG is a class of chondroitin sulfate and has physicochemical properties as shown below.
Characteristic:

white, amorphous, highly hygroscopic powder

Molecular weight:

about 15,000 to about 80,000 (as measured by high performance GPC or polyacrylamide electrophoresis)

Analysis for composition:

The composition (in terms of weight) is as follows.

| | |
|---|---|
| Galactosamine | 13 to 20 wt.% |
| Glucuronic acid | 11 to 19 wt.% |
| Fucose | 10 to 27 wt.% |
| Sulfate | 27 to 38.5 wt.% |

Analyses were conducted by the following methods to check galactosamine (hereinafter referred to as "GalN"), glucuronic acid (hereinafter referred to as "GA"), fucose (hereinafter referred to as "Fuc") and sulfate.
GalN:

White method (Carbohydrate Research, 114: 201, 1983)

GA:

Bitter-Muir method (Anal. Biochem., 4: 330, 1962)

Fuc:

4

Dische method (J. Biol. Chem., 175: 595, 1948)

Sulfate:

Dodgson & Price method (Biochem. J., 84: 106, 1962)

FGAG is a known substance disclosed in, for example, Yao Hsueh Hsueh Pao (1980, 15 (5), 263-270), Zhongyao Tongbao (1982, 7 (4), 27-29), Yaoxue Xuebao (1983, 18 (3), 203-208) and Japanese Unexamined Patent Publications Nos.10601/1988 and 128001/1988. FGAG can be easily prepared by the methods disclosed in these publications.

Stated more specifically, FGAG is prepared as follows. The body wall of a holothurian is decomposed with an alkali and further decomposed with pancreatin or like enzyme for extracion. FGAG is then isolated from the extract and purified. Examples of sea cucumbers useful in the preparation of FGAG are:

Stichopus japonicus Selenka,

Stichopus chloronoyus Brandt,

Stichopus variegatus Semper,

Holothuria pervicax Selenka,

Holothuria atra,

Holothuria argus,

Holothuria edulis,

Holothuria scabra,

Parastichopus nigripunctatus,

Thelenota ananas,

Holothuria monacaria Lesson,

Holothuria leucospilota Brandt,

Cucumaria chronhjelmi,

Cucumaria echinata,

Cucumaria frondosa Japonica,

Pentacta australis,

Paracaudina chilensis ransonneti,

Molpadia musculus,

Leptosynapta inhaerens,

Polycheira rufescens,

Synapta maculata,

Halodeima cinerascens (Brandt),

Actinopyga lacanora (Jaeger),

Actinopyga echinites (Jaeger),

Microthele nobilis (Salenka), etc.

Sea cucumbers to be used as the starting material may be a raw or dried one. Of the sea cucumbers exemplified above, Stichopus japonicus Selenka is most preferred as the starting material.

As a relative prepared by chemical conversion of FGAG, D-HG obtained by depolymerizing FGAG is especially desirable.

D-HG is prepared by dissolving FGAG or a salt thereof in water and subjecting the solution to depolymerization reaction. In the depolymerization reaction, a high-molecular sulfated polysaccharide such as heparin or the like is converted into a low-molecular sulfated polysaccharide. The reaction is conducted usually in the presence of a depolymerizing agent. Examples of useful depolymerizing agents are hydrogen peroxide, hypochlorous acid, hypobromous acid, sodium hypochlorite and like hypohalogenous acids and salts thereof; periodic acid, sodium periodate and like periodic acids and salts thereof, etc. Further, ascorbic acid, ferrous ions or the like are useful as a reaction accelerator. Alternatively, the depolymerization reaction can be effected by using radiations such as ultrasonic waves, ultraviolet rays, gamma rays or the like alone in lieu of a depolymerizing agent or in combination with the above depolymerizing agent. The most preferred depolymerization method is carried out using hydrogen peroxide as a depolymerizing agent. Hydrogen peroxide is reacted in an amount of 1 to 31 wt.%, preferably 1 to 16 wt.% in terms of hydrogen peroxide concentration. The reaction time is usually 1 to 60 hours, preferably 3 to 40 hours, and the reaction temperature ranges from room temperature to about 80°C, preferably about 40 to about 60°C. The pH range in the reaction of hydrogen peroxide is acid or neutral in the range of from 1 to 8, preferably 3 to 7. To maintain a constant pH value, hydrogen peroxide may be reacted in a buffer such as acetate buffer, phosphate buffer, Tris buffer or the like. A pH controller using a diluted sodium hydroxide may be used in the reaction. On completion of the reaction, the pH is returned to neutral range, and isolation and purification are conducted. The isolation and purification can be done, for example, by fractional precipitation using an organic solvent such as ethanol, acetone or the like; acetate such as potassium acetate, barium acetate, calcium acetate, ammonium acetate or the like; or quaternary ammonium salt such as cetyltrimethyl ammonium salt or the like. The isolation and purification can be also performed by ion exchange chromatography using resins such as DEAE-Cellulose (product of Sigma Chemical Co.), DEAE-Toyopearl (product of Tosoh Corporation), DEAE-Cellulofine (product of Chisso Corporation), Dowex-I (product of Dow Chemical Co.) or the like; by gel filtration chromatography using gels such as Sephadex G-50, Sephadex G-200 (both products of Pharmacia-LKB Biotechnology); by dialysis using Spectra/Por (product of Spectrum Medical Industries, INC.) or by ultrafiltration. These means are employed alone or in a suitable combination thereof.

The thus prepared D-HG has the following physicochemical properties.

Characteristic:
white, amorphous, highly hygroscopic powder

Molecular weight:
3,000 to 42,000 (as measured by high performance GPC)

Analysis for composition:
D-HG comprises sulfates and constituent saccharides including GalN, GA and Fuc in a molar ratio of GalN : GA : Fuc : sulfate = 1 : 0.80±0.20 : 0.85±0.15 : 3.4±0.90.

Solubility:
soluble in water but insoluble in ethanol, acetone and like organic solvents.

Specific rotation:
$[\alpha]_D^{20}$ = -55 to -73° (C = 1%)

Color reaction:
As shown below

| | |
|---|---|
| Elson-Morgan reaction | + |
| Carbazole-sulfuric acid reaction | + |
| Cysteine-sulfuric acid reaction | + |
| Orcinol-hydrochloric acid reaction | + |
| Azure A metachromasia reaction | + |

A preferred molecular weight of the D-HG is about 4,000 to about 15,000 (as measured by high performance GPC).

Shown below is the composition or constituent saccharides and the like in a form in which a salt is not formed, i.e., in free form.

| | |
|---|---|
| GalN | 18 to 24 wt.% |
| GA | 14 to 21 wt.% |
| Fuc | 13 to 20 wt.% |
| Sulfate | 31 to 44 wt.% |

As the above analysis shows, FGAG and its relative have in the molecule sulfate and carboxyl group, which react with bases to form a salt. These sulfated polysaccharides are stable in the form of a salt and are usually isolated in the form of a salt of sodium and/or potassium or the like. The salts of these sulfated polysaccharides can be also transformed into free sulfated polysaccharides by treatment with cation-exchange resins such as Dowex 50W or the like. Further, the sulfated polysaccharides, if necessary, can be converted into desired salts by conventional salt exchange commonly used. Usable as salts of sulfated polysaccharides are pharmaceutically acceptable salts including salts of potassium, sodium or like alkali metals, and salts of calcium, magnesium, barium or like alkaline earth metals, or pyridinium or like organic bases. The process for preparing FGAG and its relative will be described in detail in Reference Examples given later.

The anti-HIV agent of the invention comprising the effective components of the invention and a pharmaceutically acceptable carrier is prepared in a form varied depending on the mode of HIV treatment. The administration forms useful in the invention include tablets, capsules, powders, granules, grains, solutions, emulsions, suspensions and like coral forms, and injections, suppositories, ointment, plaster and like parenteral forms. The preparations in such form can be manufactured by conventional producing methods commonly employed by persons of ordinary skill in the art. In the production of a solid preparation for oral administration, the anti-HIV agent of the invention can be prepared by mixing the effective components of the invention with an excipient with or without addition of binders, disintegrators, lubricants, coloring agents, flavorings, perfumes, etc. and making the mixture into tablets, capsules, powders, granules, grains or the like in a conventional manner. Injectable preparations can be formulated by adding a pH-adjusting agent, buffer, stabilizer, isotonizing agent, local anesthetic and the like to the effective components, and making the mixture into intravenous, intramuscular, subcutaneous, intracutaneous or intraperitoneal injections in a conventional manner. Suppositories can be prepared by making a mixture of the effective components, base materials and when required a surfactant into a suppository in a conventional manner.

Examples of excipients useful for oral solid preparations are lactose, sucrose, starch, talc, magnesium stearate, crystalline cellulose, methyl cellulose, carboxymethyl cellulose, glycerin, sodium alginate, gum arabic, etc. Examples of the binder for oral preparations include polyvinyl alcohol, polyvinyl ether, ethyl cellulose, gum arabic, shellac, sucrose, etc. Examples of the lubricant include magnesium stearate, talc and the like. The coloring agents, disintegrators and other auxiliaries to be added include those commonly used in the art. Tablets may be coated by well-known methods.

Examples of base materials useful for suppositories include oily base materials such as macrogol, lanolin, cacao oil, fatty acid triglyceride, Witepsol (registered trade mark for the product of Dynamite Nobel) and so on.

The amount of the effective components per each unit dosage in the preparation of the invention varies with the symptoms of the patient to be given the preparation, the form of the preparation, etc. Usually a preferred amount is 10 to 200 mg in an oral preparation, 1 to 100 mg in an injection, or 10 to 100 mg in a suppository, per each unit dosage. The daily clinical dosage of the effective components of the invention also varies with the patient's age, sex, conditions and other factors but usually may be in the range of about 10 to about 1,000 mg, preferably about 50 to about 200 mg and can be given at 1 to 4 divided doses.

The anti-HIV agent of the invention is effective in preventing HIV infection, and inhibiting the incidence of, and curing, AIDS and ARC (AIDS related complex; R.R. Redfield & D. S. Burke, Science, 18, 74-87, 1988). Further, the agent can be suitably used for a long-term administration because the sulfated polysaccharide, i.e. the effective component of the agent derived from sea cucumbers, is low in cytotoxicity.

Thus the anti-HIV agent of the invention is effective in preventing normal persons from HIV infection, inhibiting the incidence of, and curing, potential symptoms latent in the carrier and treating patients of AIDS or ARC.

The present invention will be described in greater detail with reference to Reference Examples, Examples and Pharmacological Tests. The percentages in Reference Examples and Examples are all by weight.

(1) Preparation of FGAG

Reference Example 1

Preparation of FGAG from Stichopus japonicus

One kilogram of dried Stichopus japonicus was immersed in 10 ℓ of warm water overnight to swell it. The flesh was removed and homogenized. Potassium hydroxide was added in an amount to give a 1N mixture. The mixture was treated at 60°C for 100 minutes and adjusted to a pH of 8.5. After addition of 50 g of pancreatin, the mixture was stirred at 50°C for 3 hours.

After removal of impurities by centrifugation, 4.3 ℓ of ethanol was added to the residue. The mixture was allowed to stand at 4°C and the resulting precipitate was collected. The precipitate was washed with 80% ethanol, anhydrous ethanol and acetone in this sequence, and dried under reduced pressure, giving 50 g of a crude product. Fifty grams of the crude product was dissolved in 3.5 ℓ of water and the solution was centrifuged to remove the insolubles. To the supernatant were added 5% sodium chloride and 40% ethanol to give a precipitate. The precipitate was collected by centrifugation. After the precipitate was dissolved in 2.5 ℓ of water, the solution was adjusted to a pH of 10.5. To the solution was added dropwise a 30% aqueous solution of hydrogen peroxide. The mixture was decolorized in a water bath at 50°C with heating for about 3 hours. After cooling, the insolubles were removed by centrifugation. To the supernatant was added about 490 g of potassium acetate and the mixture was kept at 4°C overnight. The following day, the resulting precipitate was dissolved in 2 ℓ of water, the solution was cooled to 0°C, and the pH was adjusted to 2.8. The insolubles here removed from the solution by centrifugation. After neutralizing the supernatant, 196 g of potassium acetate was added. The mixture was allowed to stand at 4°C to give a precipitate, which was then collected by centrifugation. The precipitate was dissolved again in water to give a solution having a potassium acetate concentration of 0.5M and the solution was left overnight at 4°C. The precipitate was collected by centrifugation, washed with 40% methanol and dissolved in 1 ℓ of water. To the solution were added 5% sodium chloride and 40% ethanol to give a precipitate. The precipitate was collected by centrifugation, washed with 80% methanol, anhydrous ethanol and acetone in this sequence, and dried under reduced pressure, giving 17 g of a FGAG sodium/potassium salt. The physicochemical constants of the salt are as follows.

Molecular weight:
55,000 (as determined by high performance GPC)
Analysis for composition:     As shown below

| GalN: | 20.0 % |
| GA: | 18.6 % |
| Fuc: | 17.2 % |
| Sulfate: | 36.6 % |
| Na: | 6.2 % |
| K: | 7.4 % |

Reference Example 2

Preparation of FGAG from Holothuria leucospilota Brandt

Using Holothuria leucospilota Brandt, FGAG derived therefrom was prepared by the same procedure as in Reference Example 1.

Molecular weight:     45,000 to 55,000 (as determined by polyacrylamide electrophoresis)
Analysis for composition:     As shown below

| GalN: | 16.0 % |
| GA: | 16.3 % |
| Fuc: | 12.3 % |
| Sulfate: | 31.0 % |

Reference Example 3

Preparation of FGAG from Cucumaria frondosa Japonica

Using Cucumaria frondosa Japonica, FGAG derived therefrom was prepared by the same procedure as in Reference Example 1.

Molecular weight:
60,000 to 70,000 (as determined by polyacrylamide electrophoresis)
Analysis for composition:     As shown below

| GalN: | 18.0 % |
| GA: | 11.1 % |
| Fuc: | 13.7 % |
| Sulfate: | 32.0 % |

(2) Preparation of D-HG

Reference Example 4

Ten grams of the FGAG sodium/potassium salt obtained in Reference Example 1 was dissolved in 75 ml of water and 25 ml of a 30% aqueous solution of hydrogen peroxide was added. While maintaining the solution at a pH of about 7 with a diluted sodium hydroxide solution using a pH controler, the solution was heated at 60°C for 12 hours. After cooling, 2% sodium chloride and 40% ethanol were added to give a precipitate. The precipitate was collected by centrifugation, washed with 80% ethanol, anhydrous ethanol and acetone in this sequence, and dried under reduced pressure, giving 7.15 g of a D-HG sodium/potassium salt.

### Reference Example 5

A 6.95 g quantity of a D-HG sodium/potassium salt was prepared by the same procedure as in Reference Example 4 with the exception of treatment with hydrogen peroxide for 24 hours.

### Reference Example 6

A D-HG sodium/potassium salt was prepared by the same procedure as in Reference Example 4 with the exception of conducting the reaction while maintaining the pH of about 4 with a diluted alkali solution. Yield 6.4 g.

### Reference Example 7

Ten grams of the FGAG sodium/potassium salt obtained in Reference Example 1 was dissolved in 83.3 ml of a 0.2M phosphate buffer (pH 7.0). To the solution was added 16.7 ml of a 30% aqueous solution of hydrogen peroxide. The mixture was treated at 60°C for 12 hours. After cooling, 2% sodium chloride and 40% ethanol were added to give a precipitate. The precipitate was collected by centrifugation, washed with 80% ethanol, anhydrous ethanol and acetone in this sequence, and dried under reduced pressure, giving 7.18 g of a D-HG sodium/potassium salt.

### Reference Example 8

A D-HG sodium/potassium salt was prepared by the same procedure as in Reference Example 7 with the exception of conducting the reaction using a 0.2M acetate buffer (pH 3.5). Yield 7.05 g.

### Reference Examples 9 and 10

Two grams of the FGAG sodium/potassium salt obtained in Reference Example 1 was dissolved in 15 ml of water. To the solution was added 5 ml of a 30% aqueous solution of hydrogen peroxide, and the mixture was treated at 60°C for 14 hours (Reference Example 9) or 40 hours (Reference Example 10). After cooling, the mixture was adjusted to a pH of 7 to 8 and thoroughly dialyzed against water using Spectra/por 3. The mixture was lyophilized and dried under reduced pressure. In this way, 1.62 g and 1.76 g of D-HG sodium/potassium salts were prepared.

### Reference Example 11

Two grams of the FGAG sodium/potassium salt obtained in Reference Example 1 was dissolved in 16.7 ml of water. To the solution was added 3.3 ml of a 30% aqueous solution of hydrogen peroxide and the mixture was treated at 45°C for 24 hours. After cooling, the mixture was returned to a pH of about 7 after which 2% sodium chloride and 40% ethanol were added to provide a precipitate. The precipitate was collected by centrifugation, washed with 80% ethanol, anhydrous ethanol and acetone in this sequence, and dried under reduced pressure, giving 1.41 g of a D-HG sodium/potassium salt.

### Reference Examples 12 to 15

Two grams of the FGAG sodium/potassium salt obtained in Reference Example 1 was dissolved in 15 ml of water. To the solution was added 5 ml of a 30% aqueous solution of hydrogen peroxide, and the mixture was treated at 60°C for 4, 8, 12 or 24 hours. After cooling, the mixture was adjusted to a pH of 7 to 8 and fully dialyzed against water using Spectra/por 3. The same treatment as in Reference Example 11 followed. In this way, 1.42 g, 1.35 g, 1.35 g and 1.2 g of D-HG sodium/potassium salts were produced.

Reference Examples 16 and 17

Two grams of the FGAG sodium/potassium salt obtained in Reference Example 1 was dissolved in 14.7 ml of water. To the solution was added 5.3 ml of a 30% aqueous solution of hydrogen peroxide. The mixture was treated at 45°C for 14 hours or 40 hours. After cooling, the mixture was returned to a pH of about 7 after which 2% sodium chloride and 40% ethanol were added to give a precipitate. The same treatment as in Referece Examples 9 to 11 followed. In this way, 1.64 g and 1.62 g of D-HG sodium/potassium salts were prepared.

Reference Example 18

Two grams of the FGAG sodium/potassium salt obtained in Reference Example 1 was dissolved in 30 ml of water and treated for 12 hours in the same manner as in Reference Example 11. The solution was fractionated with a solution of sodium chloride on a Sephadex G-50T column (product of Pharmacia-LKB Biotechnology). While monitoring uronic acid, peaks were divided into three. The eluate obtained last was collected, fully dialyzed against water, lyophilized and dried under reduced pressure, giving 0.2 g of a D-HG sodium salt.

Reference Example 19

A 0.5 g quantity of the D-HG sodium/potassium salt obtained in Reference Example 11 was fractionated in the same manner as in Reference Example 18, giving 0.18 g of a D-HG sodium salt.

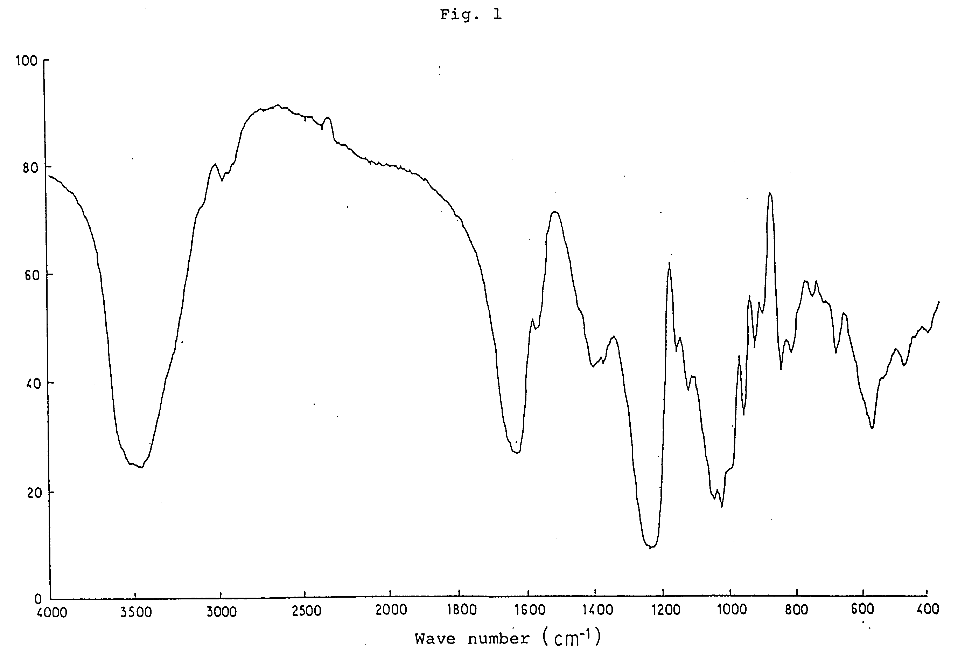

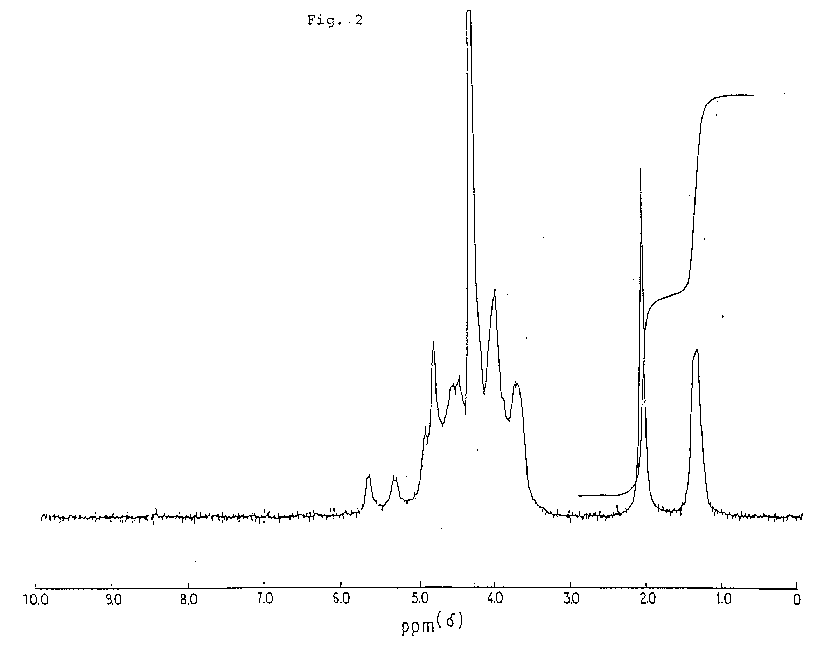

Fig. 1 shows an infrared absorption spectrum of the D-HG sodium salt (as measured with KBr tablet) and Fig. 2 a proton nuclear magnetic resonance spectrum (in $D_2O$, 90MHz, 70°C) thereof.

Reference Example 20

Two grams of the FGAG sodium/potassium salt obtained in Reference Example 1 was dissolved in 16.7 ml of water, and 3.3 ml of a 30% aqueous solution of hydrogen peroxide was added. The mixture was treated at 45°C for 5 hours. After cooling, the mixture was returned to a pH of about 7 after which 2% sodium chloride and 40% ethanol were added to give a precipitate. The precipitate was collected by centrifugation, washed with 80% ethanol, anhydrous ethanol and acetone in this sequence, and dried under reduced pressure, giving a D-HG sodium/potassium salt. Yield 1.60 g.

Reference Example 21

Two grams of the FGAG sodium/potassium salt obtained in Reference Example 1 was treated for 9 hours by the same method as in Reference Example 20, giving 1.54g of a D-HG sodium/potassium salt.

Reference Example 22

Two grams of the FGAG sodium/potassium salt obtained in Reference Example 1 was treated for 12 hours by the same method as in Reference Example 20, giving 1.52 g of a D-HG sodium/potassium salt.

Reference Example 23

One gram of the FGAG sodium/potassium salt obtained in Reference Example 1 was dissolved in 8.7 ml of 0.2M phosphate buffer (pH 7.0). To the solution was added 1.3 ml of a 30% aqueous solution of hydrogen peroxide and the mixture was treated at 60°C for 3 hours. After cooling, 5% sodium chloride and 66% ethanol were added to the mixture to give a precipitate. The precipitate was collected by centrifugation, washed with 80% ethanol, anhydrous ethanol and diethyl ether in this sequence, and dried under reduced pressure, giving a D-HG sodium/potassium salt. The obtained salt was dissolved in 10 ml of 20mM Tris-HCl buffer (pH 7.0) and admixed with DEAE-Toyopearl (product of Tosoh Corporation) thoroughly equilibrated with the buffer. Elution was conducted in the buffer with a linear concentration gradient of sodium chloride (0 to 1 M). While monitoring uronic acid, peak fractions were collected and precipitation occurred with addition of a two-fold amount of ethanol. The precipitate was collected by centrifugation, washed with 80% ethanol, anhydrous ethanol and diethyl ether in this order and dried under reduced pressure, giving a D-HG sodium salt. Yield 0.39 g.

Table 1 shows physicochemical properties of D-HGs' obtained in Reference Examples 4 to 23.

Table 1

| Ref. Ex. | MW x 10^3 | $[\alpha]_D^{20}$ | Composition | | | | | | Molar ratio | | | |
|---|---|---|---|---|---|---|---|---|---|---|---|---|
| | | | GalN | Fuc | GA | Sul* | Na | K | GalN : | GA : | Fuc : | Sul* |
| 4 | 10.2 | -71.2 | 21.7 | 16.7 | 16.5 | 29.7 | 7.1 | 6.4 | 1 : | 0.70 : | 0.84 : | 2.5 |
| 5 | 9.7 | -67.1 | 19.8 | 15.8 | 15.1 | 30.2 | 7.2 | 7.9 | 1 : | 0.70 : | 0.87 : | 2.8 |
| 6 | 14.1 | -71.5 | 20.2 | 17.6 | 15.6 | 33.1 | 6.2 | 6.7 | 1 : | 0.71 : | 0.95 : | 3.0 |
| 7 | 4.7 | -55.4 | 19.8 | 15.1 | 13.3 | 31.4 | 8.2 | 4.8 | 1 : | 0.62 : | 0.83 : | 2.9 |
| 8 | 6.5 | -61.2 | 20.6 | 15.6 | 15.8 | 32.8 | 7.8 | 4.4 | 1 : | 0.71 : | 0.83 : | 2.9 |
| 9 | 12.0 | -70.8 | 18.6 | 16.5 | 17.1 | 38.6 | 4.7 | 6.1 | 1 : | 0.85 : | 0.97 : | 3.8 |
| 10 | 7.8 | -68.0 | 17.5 | 13.1 | 16.1 | 37.1 | 5.4 | 6.4 | 1 : | 0.85 : | 0.82 : | 3.9 |
| 11 | 13.4 | -70.1 | 17.1 | 14.6 | 14.6 | 32.7 | 8.1 | 5.5 | 1 : | 0.79 : | 0.93 : | 3.5 |
| 12 | 14.1 | -69.6 | 19.9 | 14.3 | 14.8 | 34.0 | 7.6 | 5.2 | 1 : | 0.69 : | 0.79 : | 3.1 |
| 13 | 8.6 | -66.3 | 20.8 | 15.1 | 14.3 | 34.8 | 8.5 | 5.5 | 1 : | 0.64 : | 0.79 : | 3.1 |

Table 1 (Continued)

| Ref. Ex. | MW x $10^3$ | $[\alpha]_D^{20}$ | Composition | | | | | | Molar ratio GalN : GA : Fuc : Sul* | | | |
|---|---|---|---|---|---|---|---|---|---|---|---|---|
| | | | GalN | Fuc | GA | Sul* | Na | K | | | | |
| 14 | 7.5 | −66.0 | 17.8 | 13.2 | 13.2 | 32.9 | 6.4 | 5.1 | 1 : 0.68 : 0.81 : 3.4 | | | |
| 15 | 5.6 | −64.8 | 20.1 | 14.7 | 13.0 | 33.4 | 4.1 | 5.1 | 1 : 0.60 : 0.80 : 3.0 | | | |
| 16 | 10.8 | −72.2 | 18.1 | 14.8 | 16.7 | 37.1 | 5.0 | 6.5 | 1 : 0.85 : 0.89 : 3.8 | | | |
| 17 | 6.6 | −68.4 | 17.3 | 12.8 | 15.9 | 35.3 | 5.6 | 6.7 | 1 : 0.85 : 0.81 : 3.7 | | | |
| 18 | 10.2 | −68.2 | 19.1 | 15.8 | 14.4 | 32.9 | 8.6 | 0 | 1 : 0.69 : 0.90 : 3.1 | | | |
| 19 | 7.7 | −67.5 | 17.6 | 13.9 | 14.1 | 32.1 | 4.4 | 0 | 1 : 0.74 : 0.86 : 3.3 | | | |
| 20 | 41.4 | −70.7 | 19.8 | 14.7 | 15.8 | 29.9 | 5.9 | 5.5 | 1 : 0.73 : 0.81 : 2.7 | | | |
| 21 | 24.3 | −72.6 | 18.0 | 15.9 | 16.6 | 33.4 | 6.2 | 5.2 | 1 : 0.85 : 0.96 : 3.4 | | | |
| 22 | 20.1 | −71.1 | 18.3 | 14.4 | 14.8 | 33.0 | 7.5 | 5.0 | 1 : 0.75 : 0.86 : 3.3 | | | |
| 23 | 12.8 | −62.4 | 19.3 | 14.5 | 16.2 | 34.3 | 10.7 | 0 | 1 : 0.78 : 0.81 : 3.3 | | | |

Note: In Table 1, the molecular weight (MW) was determined by high performance gel permeation chromatography and the other property values were determined as described hereinbefore. Sul* stands for sulfate.

The sulfated polysaccharides obtained in Reference Examples 1 to 23 showed single spots in electrophoresis (Dietrich. C. P., J. Chromatogr., 130, 299 (1977)).

12

Preparation of anti-HIV agent

## Example 1

Injection preparation

The FGAG sodium/potassium salt prepared in Reference Example 1 was dissolved in distilled water for injection to give a 5% aqueous solution. A 50 mg quantity of the solution containing the sulfated polysaccharide derived from the sea cucumber was filled into a vial to perform lyophilization. A 2 ml quantity of physiological saline was added as a solvent.

## Example 2

Injection preparation

An injection preparation was prepared according to the formulation as shown below.

| FGAG sodium/potassium salt (Reference Example 1) Physiological saline | 40 mg q.s. |
|---|---|
| Per ampule | 2 ml |

## Example 3

Tablet

Tablets were prepared according to the formulation as shown below.

| Sodium salt of D-HG (Reference Example 19) Corn starch Carboxymethyl cellulose Polyvinyl pyrrolidone Magnesium stearate | 10 mg 65 mg 20 mg 3 mg 2 mg |
|---|---|
| Per tablet | 100 mg |

## Example 4

Suppository

As suppository was prepared according to the formulation as shown below.

| D-HG sodium/potassium salt (Reference Example 17) Witepsol W-35 (Product of Dynamite-Nobel AG) | 50 mg 950 mg |
|---|---|
| Per suppository | 1000 mg |

Pharmacological Test

(1) Anti-HIV activity

MT-4 cells (Miyoshi I. et al, Gann Monogr., 28, 219-228(1982)) were adjusted to provide a $2 \times 10^5$ cells/ml medium (RPMI-1640, +10% FCS). To 500 $\mu$l of the medium were added 50 $\mu$l of a solution of each of sea cucumber-derived sulfated polysaccharides obtained in Reference Examples 1, 19 and 23 in a varying concentration. Two hours after the addition, the medium was infected with HIV in a $5 \times 10^3$ Pfu/well ratio. On the 4th day after the infection, a smear of MT-4 cells was prepared and the HIV antigen positive cells were investigated by fluorescent antibody method (Harada S., et al., Science, 229, 563-566 (1985); Takeuchi, Y., et al., Jpn. J. Cancer Res. (Gann), 78, 11-15 (1987)).

Tables 2, 3 and 4 below show the percentages (%) of the HIV-infected cells (HIV antigen positive cells).

Table 2

| FGAG concentration (Ref. Ex. 1) (μg/ml) | 10 | 1 | 0.1 | 0 control |
|---|---|---|---|---|
| Percentage (%) | 0 | 15 | 95 | 95 |

Table 3

| D-HG concentration (Ref. Ex. 19) (μg/ml) | 30 | 3 | 0.3 | 0.03 | 0 control |
|---|---|---|---|---|---|
| Percentage (%) | 0 | 8 | 25 | 95 | 95 |

Table 4

| D-HG concentration (Ref. Ex. 23) (μg/ml) | 10 | 1 | 0.1 | 0 control |
|---|---|---|---|---|
| Percentage (%) | 0 | 10 | 95 | 95 |

The results show that the anti-HIV agents of the present invention effectively prevent HIV infection.

(2) Cytotoxicity

The sulfated polysaccharides derived from sea cucumbers were tested for cytotoxicity in the same manner as in item (1). Stated more specifically, MT-4 cells adjusted to give a $1 \times 10^5$ cells/ml medium were incubated for 4 days in the presence of each of the sea cucumber-derived sulfated polysaccharides obtained in Reference Examples 1 and 19 in a varying concentration. On the 4th day after incubation, the number of cells was counted to evaluate the cytotoxicity in terms of indexes calculated at the listed concentrations when the control had 100 cells. Tables 5 and 6 below show the results.

Table 5

| FGAG concentration (Ref. Ex. 1) (μg/ml) | 100 | 10 | 1 | 0 control |
|---|---|---|---|---|
| Index | 77 | 105 | 104 | 100 |

Table 6

| D-HG concentration (Ref. Ex. 19) (μg/ml) | 100 | 30 | 3 | 0.3 | 0 control |
|---|---|---|---|---|---|
| Index | 83 | 90 | 102 | 104 | 100 |

The results show that the anti-HIV agents of the present invention have no significant cytotoxicity.

(3) Acute toxicity

The FGAG sodium/potassium salt prepared in Reference Example 1 was subjected to an acute toxicity test in mice. The test results show that the salt is 337.9 mg/Kg (i.p.), and 335.2 mg/Kg (i.v.) in $LD_{50}$, and

that the salt is remarkably safe.

**Claims**

1.  Use of a sulfated polysaccharide derived from a sea cucumber or a pharmaceutically acceptable salt thereof for the preparation of a pharmacological composition for treating diseases caused by HIV infection, said sulfated polysaccharide consisting of galactosamine, glucuronic acid, fucose and sulfate.

2.  Use according to claim 1 wherein said sulfated polysaccharide has the following physicochemical properties:
    Characteristic:                   white, amorphous, highly hygroscopic powder
    Molecular weight:           about 15,000 to about 80,000 (as measured by high performance GPC or polyacrylamide electrophoresis)
    Composition (in terms of weight):

| | |
|---|---|
| galactosamine | 13 to 20 wt.% |
| glucuronic acid | 11 to 19 wt.% |
| fucose | 10 to 27 wt.% |
| sulfate | 27 to 38.5 wt.% |

3.  Use according to claim 1 wherein said sulfated polysaccharide has the following physicochemical properties:
    Characteristic:     white, amorphous, highly hygroscopic powder
    Molecular weight:  3,000 to 42,000 (as measured by high performance GPC)
    Molar ratio:

    galactosamine : glucuronic acid : fucose : sulfate = 1 : 0.80±0.20 : 0.85±0.15 : 3.4±0.90.
    Solubility:          soluble in water but insoluble in ethanol, acetone and like organic solvents.
    Specific rotation:  $[\alpha]_D^{20}$ = -55 to -73° (C = 1%)
    Color reaction:

| | |
|---|---|
| Elson-Morgan reaction | + |
| Carbazole-sulfuric acid reaction | + |
| Cysteine-sulfuric acid reaction | + |
| Orcinol-hydrochloric acid reaction | + |
| Azure A metachromasia reaction | + |

**Patentansprüche**

1.  Verwendung eines sulfatierten Polysaccharids, das aus einer Seegurke stammt, oder eines pharmazeutisch verträglichen Salzes davon zur Herstellung eines Arzneimittels für die Behandlung von Krankheiten, die durch eine HIV-Infektion verursacht werden, wobei das sulfatierte Polysaccharid aus Galactosamin, Glucuronsäure, Fucose und Sulfat besteht.

2.  Verwendung nach Anspruch 1, wobei das sulfatierte Polysaccharid die folgenden physikalisch-chemischen Eigenschaften aufweist:
    Merkmale:                 weißes, amorphes, stark hygroskopisches Pulver
    Molekulargewicht:         etwa 15 000 bis etwa 80 000 (gemessen durch Hochleistungs-GPC oder Polyacrylamid-Elektrophorese)
    Zusammensetzung (bezogen auf Gewicht):

**EP 0 410 002 B1**

| Galactosamin | 13 bis 20 Gew.-% |
|---|---|
| Glucuronsäure | 11 bis 19 Gew.-% |
| Fucose | 10 bis 27 Gew.-% |
| Sulfat | 27 bis 38,5 Gew.-%. |

3. Verwendung nach Anspruch 1, wobei das sulfatierte Polysaccharid die folgenden physikalisch-chemischen Eigenschaften aufweist:

Merkmale:    weißes, amorphes, stark hygroskopisches Pulver
Molekulargewicht:    3 000 bis 42 000 (gemessen durch Hochleistungs-GPC)
Molares Verhältnis:

Galactosamin : Glucuronsäure : Fucose : Sulfat = 1 : 0,80 ± 0,20 : 0,85 ± 0,15 : 3,4 ± 0,90

Löslichkeit:    Löslich in Wasser, jedoch unlöslich in Ethanol, Aceton und ähnlichen organischen Lösungsmitteln

Spezifische Drehung:    $[\alpha]_D^{20}$ = -55 bis -73° (C = 1%)
Farbreaktion:

| Elson-Morgan-Reaktion | + |
|---|---|
| Carbazol-Schwefelsäure-Reaktion | + |
| Cystein-Schwefelsäure-Reaktion | + |
| Orcinol-Salzsäure-Reaktion | + |
| Azur A-Metachromasie-Reaktion | + |

**Revendications**

1. Utilisation d'un polysaccharide sulfaté dérivant d'un concombre de mer, ou utilisation d'un de ses sels pharmaceutiquement acceptables, pour la préparation d'un composition pharmacologique pour le traitement de maladies causées par l'infection par l'HIV, ledit polysaccharide sulfaté consistant en galactosamine, acide glucuronique, fucose et sulfate.

2. Utilisation selon la revendication 1, dans laquelle ledit polysaccharide sulfaté a les propriétés physicochimiques suivantes :
   - Caractéristiques : poudre blanche, amorphe, fortement hygroscopique
   - Poids moléculaire : environ 15.000 à environ 80.000 (mesuré par CPV haute performance, ou par électrophorèse sur polyacrylamide)
   - Composition (en poids) :

| • Galactosamine | 13 à 20 % en poids |
|---|---|
| • Acide glucuronique | 11 à 19 % en poids |
| • Fucose | 10 à 27 % en poids |
| • Sulfate | 27 à 38,5 % en poids |

3. Utilisation selon la revendication 1, dans laquelle ledit polysaccharide sulfaté a les propriétés physicochimiques suivantes :
   - Caractéristiques : poudre blanche, amorphe, fortement hygroscopique
   - Poids moléculaire : 3.000 à 42.000 (mesuré par CPV haute performance)
   - Ratio molaire :
     Galactosamine/Acide glucuronique/Fucose/Sulfate = 1 / 0,80 (± 0,20) / 0,85 (± 0,15) / 3,4 (± 0,90)
   - Solubilité : soluble dans l'eau, mais insoluble dans l'éthanol, l'acétone, et les solvants organiques similaires
   - Pouvoir rotatoire : $[\alpha]_D^{20}$ = -55 à -73° (C = 1 %)
   - Réactions colorées :

16

| Réaction d'Elson-Morgan | + |
| Réaction carbazole-acide sulfurique | + |
| Réaction cystéine-acide sulfurique | + |
| Réaction orcinol-acide chlorhydrique | + |
| Réaction au Métachromasia Azur A | + |

Fig. 1

EP 0 410 002 B1

Fig. 2